# EUROPÄISCHE PATENTSCHRIFT

(11) **EP 0 464 976 B1**
(45) Veröffentlichungstag und Bekanntmachung des Hinweises auf die Patenterteilung: **27.12.1995**
(21) Anmeldenummer: 91250168.1
(22) Anmeldetag: 24.06.1991
(51) Int. Cl.: C12N 1/00, C12P 1/00

(54) **Verfahren zur Gewinnung lipophiler Zellinhaltsstoffe**
Isolation of lipophilic cell components
Procédé pour l'extraction de la matière lipophile de cellules

(30) Priorität: 02.07.1990 DD 342408
(43) Veröffentlichungstag der Anmeldung: 08.01.1992
(73) Patentinhaber: Buna Sow Leuna Olefinverbund GmbH, 06258 Schkopau (DE)
(72) Erfinder: Mayer, Torsten, O-4070 Halle (DE); Kunze, Hartmut, Dr., O-4020 Halle (DE); Schaffer, Jürgen, Dr., O-4090 Halle (DE); Rauchstein, Klaus-Dieter, Dr., O-4090 Halle (DE); Hummel, Kathleen, O-4090 Halle (DE); Müller, Heidi, O-4020 Halle (DE)

(56) Entgegenhaltungen:
- DD-A- 151 007

## Beschreibung

Die Erfindung betrifft ein Verfahren zur Gewinnung lipophiler Zellinhaltsstoffe aus mikrobiellen Biomassen, insbesondere aus auf Abwässern kultivierten Mischbiozönosen.

Mikroorganismen, die auf kohlenwasserstoffhaltigen Substraten gezüchtet werden, enthalten trotz sorgfältiger Anreicherung und Abtrennung immer beträchtliche Mengen an biogenen lipophilen Zellinhaltsstoffen.
Vor der Verwendung der aus den Mikroorganismen hergestellten Biomassen oder vor der Weiterverarbeitung, z. B. zur Herstellung von Eiweißisolaten, müssen die lipophilen Zellinhaltsstoffe entfernt werden.
Dazu sind verschiedene Verfahren bekannt, die zum großen Teil über eine Trocknung und anschließende Extraktion der festen, pulverförmigen Biomassen ablaufen. Als Lösungsmittel werden demgemäß Kohlenwasserstoffe und polare Lösungsmittel vorgeschlagen. Zur Erzeugung von Futtereiweiß (DD-WP 230556) wird die sprühgetrocknete Biomasse zweistufig mit Ethanol, Wasser, Benzin extrahiert. In der Patentschrift DD-WP 145406 wird ein anderes Lösungsmittelgemisch mit Benzin, Methanol und Dimethylformamid zur Reinigung mikrobieller Biomassen von lipophilen Verunreinigungen verwendet. Je nach Verwendungszweck der zu gewinnenden Zellinhaltsstoffe besteht ein breites Spektrum an 25 Lösungsmittel - Gemischen und der prozentualen Zusammensetzung der einzelnen Komponenten. Folgende bekannte Lösungen zur Extraktion sprühgetrockneter Biomassen seien an dieser Stelle noch genannt:
- Die Extraktion mit Methanol bei 20 °C bis 60 °C wobei ein Wassergehalt der Biomasse bis zu 10 Masseanteilen in % möglich ist (DD-WP 151676).
- Eine Extraktion mit Benzen-Ethanol-Wasser (DD-WP 156125), sowie die Extraktion mit methanolischer Ammoniaklösung, beschrieben in der Patentschrift DE-PS 2633666.

Da die Extraktion der lipophilen Zellinhaltsstoffe auch einen direkten Einfluß auf die Zerstörung von Zellwandstrukturen hat und somit die Exraktion spezifischer Zellinhaltsstoffe erleichtert, wurden andere Möglichkeiten zum Zellaufschluß gesucht. Die Konditionierung der Biomasse durch Hitzebehandlung bei 105 °C bis 160 °C (DE-PS 3312166, DD-WP 221755) vor einer Extraktion mit Lösungsmitteln ist ein Bindeglied zwischen der Extraktion getrockneter und feuchter Biomassen.
Die Extraktion wässriger Mikroorganismensuspensionen nach entsprechender Separation bzw. Aufkonzentrierung, ist das zweite große Feld zur Entfernung bzw. Gewinnung lipophiler Zellinhaltsstoffe.

Hierbei spielt die Kohlendioxid - Extraktion unter überkritischen Bedingungen (120 °C, 40,5 MPa) eine besondere Rolle (DD-WP 212746). Diese Extraktionsmethode läßt einen maximalen Wassergehalt bis zu 90 Massenanteilen in % zu und ermöglicht die gezielte Gewinnung von Zellinhaltsstoffen. In der Patentschrift DD-WP 155788 ist ein Verfahren zur Entfernung von Kohlenwasserstoffen und biogenen Inhaltsstoffen mit Feststoffgehalten von 17 Masseanteilen in % Biomassen und 80 Masseanteilen in % Wasser durch die Kopplung von Druck und Entspannung beschrieben. Betrachtet man alle Verfahren, ist die Einsatzcharakteristik entscheidend für ihre Ausführung. Entweder handelt es sich um Reinigungsverfahren, die eine möglichst vollständige Entfernung störender Verbindungen erfordert (lipophile Zellinhaltsstoffe) oder es geht um die zielgerichtete Gewinnung einzelner Verbindungen und Stoffgruppen aus der Gruppe dieser biogenen Verbindungen. Allen genannten Verfahren ist gemeinsam, daß erhebliche Aufwendungen zur Wiederbeschaffung oder Rückgewinnung der Extraktionsmittel aber auch darüber hinaus hohe Energieaufwendungen erforderlich sind. Diese hohen Aufwendungen schließen den Einsatz der genannten Verfahren zur Aufarbeitung sekundärer Abfallbiomassen aus wirtschaftlichen Gründen aus.

Das Ziel der Erfindung ist ein leistungsfähiges Verfahren zur Lösungsmittelextraktion von Biomassen, das eine weitgehende Entfernung und Gewinnung lipophiler Zellinhaltsstoffe mit geringem Aufwand ermöglicht und die Verwendung sekundärer Abfallbiomassen zur Wertstoffgewinnung gestattet.

Der Erfindung liegt die Aufgabe zu Grunde, ein Verfahren zur Gewinnung von lipophilen Zellinhaltsstoffen aus mikrobiellen Biomassen, insbesondere aus auf Abwässern kultivierten Mischbiozönosen zu entwickeln.
Erfindungsgemäß wird die Aufgabe dadurch gelöst, daß die mikrobielle Biomasse mit einem Wasseranteil bis zu 90 Massenanteilen in % durch eine fest/flüssig Extraktion mit einem Keton der Siedepunkte von 56 °C bis 155 °C bei einer Temperatur zwischen 40 °C und 85 °C aufgearbeitet wird.
Dazu kann die mikrobielle Biomasse durch bekannte mechanische oder andere Verfahren auf einen Wasseranteil bis zu 85 Masseanteilen in % vorher aufkonzentriert werden. Das Extraktionsverfahren wird in einem Rührwerksextraktor bei einem Masseverhältnis des Keton zur mikrobiellen Biomasse von 2,5 : 1 bis 5 : 1 durchgeführt.
Der Extraktion nachfolgend wird nach Abtrennen der Miscella dem gewonnenen Gemisch aus Keton, Wasser und lipophile Zellinhaltsstoffe im Masseverhältnis 1 : 2 bis 1 : 3 eine gesättigte Lösung eines Alkalisalzes zugesetzt und die Phase Keton - lipophile Zellinhaltsstoffe in bekannter Weise aufkonzentriert. Das Verfahren läßt sich besonders effektiv dann durchführen, wenn als Extraktionsmittel Aceton eingesetzt wird und die gesättigte Lösung eines Alkalisalzes eine gesättigte Lösung aus Natriumchlorid darstellt.

Die Extraktion der wasserhaltigen, mikrobiellen Biomasse mit einem Keton erbringt nach der mechanischen Abtrennung der Miscella ein homogenes Mehrstoffsystem aus Wasser, Keton und lipophilen Zellinhaltsstoffen. Gegenüber herkömmlichen Verfahren, die durch thermische und/oder mechanische Verfahren eine Auftrennung des Systems erreichten, wurde gefunden, daß die Zugabe einer gesättigten Alkalisalzlösung eine Trennung des Systems in einzelne Komponenten ermöglicht und somit zu einer wesentlichen Reduzierung des Energieverbrauchs und der apparativen Aufwendungen beiträgt. Dabei scheidet sich die lipidhaltige Ketonphase als leichtere Trennphase ab.
Die Erfindung soll nachstehend an zwei Ausführungsbeispielen erläutert werden. Dabei wird in einer labortechnischen, diskontinuierlich arbeitenden Extraktionsanlage mit einem Volumen von 1 : 1 eine auf Abwasser kultivierte Mischbiozönose extrahiert. Der Anteil der gesamtextrahierbaren lipophilen Zellinhaltsstoffe beträgt 40 mg/g Trockensubstanz an Biomasse.

### Beispiel 1:

100 g einer mechanisch aufkonzentrierten, feuchten Biomasse mit einem Wassergehalt von 90 Masseanteilen in % wird in einem Rührwerksextraktor mit einer Menge von 500 ml Aceton vermischt und bei einer Temperatur von 56 °C 30 min extrahiert.
Nach der mechanischen Abtrennung der Miscella werden dem Wasser-Aceton - lipophile Zellinhaltsstoffe Gemisch (99,/0,1 Masseanteilen in %) mit einem Wassergehalt kleiner 2 Volumenanteilen in % und 90 Volumenanteilen in % eines Wasser-Acetongemisches (42/58 Masseanteile in %).
Nach der Entfernung des Acetons aus der Aceton - lipophile Zellinhaltsstoffe - Phase wurden 20 Masseanteile in % der gesamtextrahierbaren lipophilen Zellinhaltsstoffe erhalten.

### Beispiel 2:

100 g einer feuchten Biomasse mit einem Wassergehalt von 92 Masseanteilen in % werden mit einer Menge von 275 ml Aceton vermischt und bei einer Temperatur von 56 °C 25 min extrahiert. Nach der mechanischen Abtrennung der Miscella werden dem Wasser-Aceton - lipophile Zellinhaltsstoffe - System 150 ml einer 26,4 %igen Natriumchloridlösung unter Rühren zugegeben.
Die in Beispiel 1 beschriebene Phasentrennung läßt die entstandenen Phasen wie folgt charakterisieren:
- Phase 1:: 8 Volumenanteile in % Aceton - lipophile Zellinhaltsstoffe (99,75/0,25 Masseanteile in %) mit einem Restwassergehalt kleiner 1 Masseanteil in %.
- Phase 2:: 92 Volumenanteile Wasser-Aceton-Gemisch (49/51 Masseanteile in %)
Es konnten 25 Masseanteile in % der geamtextrahierbaren lipophilen Zellinhaltsstoffe nach Entfernung des Acetons erhalten werden.

## Patentansprüche

1. Verfahren zur Gewinnung lipophiler Zellinhaltsstoffe aus mikrobiellen Biomassen, insbesondere auf Abwässern kultivierten Mischbiozönosen, die mit einem Wasseranteil bis zu 92 Masseanteilen in % bei 40 °C bis 85 °C mit einem Keton vom Siedepunkt zwischen 56 °C bis 155 °C bei einem Masseverhältnis von Keton zu mikrobieller Biomasse von 2,5 : 1 bis 5 : 1 extrahiert werden, dadurch gekennzeichchnet, daß nach dem Abtrennen der Miscella dem Gemisch Keton - Wasser - lipophile Zellinhaltsstoffe eine gesättigte Lösung an Alkalisalzen im Verhältnis 1 : 2 bis 1 : 3 zugesetzt und das Gemisch Keton - lipophile Zellinhaltsstoffe in bekannter Weise aufkonzentriert wird.

2. Verfahren nach Anspruch 1 dadurch gekennzeichnet, daß eine gesättigte Lösung aus Natriumchlorid verwendet wird.

## Claims

1. Process to obtain lipophile cell contents consisting of microbial biomasses, especially of mixed populations of microorganisms cultivated on waste waters which are extracted having a water proportion of up to 92 parts by mass in % at 40 to 85 °C with a ketone having a boiling point between 56 and 155 °C at a mass ratio of ketone and microbial biomass of 2.5 : 1 to 5 : 1, whereas after separation of the miscella, a saturated solution of alkali salts at a ratio of 1 : 2 to 1 : 3 is added to the ketone - water - lipophile cell contents - mixture, and the ketone - lipophile cell contents - mixture is concentrated in a common way as known.

2. Process according to claim 1, whereas a saturated solution of sodium chloride is applied.

## Revendications

1. Procedé pour l'obtention de matières lipophiles cellulaires contenues à partir de masses biologiques microbiennes, en particuliers des mélanges de cultures biologiques cultivées en eau usée, qui sera extraite au moyen d'une quantité d'eau à 92 quantité de masse en pourcentage, sous 40°C à 85°C avec un cétone au point de réchauffement entre 56°C et 155°C avec un rapport de cétone avec la masse biologique microbienne de 2,5 : 1 à 5 : 1, en cela caractérisé qu'après la séparation du micelle du mélange cétone - eau - matières lipophiles cellulaires contenues, on ajoutera une solution saturé en sel alcalin d'un rapport de 1 : 2 à 1 : 3 et le mélange cétone - matières lipophiles cellulaires contenues sera concentré de la façon connue.

2. Le procédé est caractérisé d'après la revendication 1, en cela, qu'il sera employé une solution saturée de chlorure de sodium.
